# FASCICULE DE BREVET EUROPEEN

(11) **EP 1 113 863 B1**
(45) Date de publication et mention de la délivrance du brevet: **19.01.2005**
(21) Numéro de dépôt: 00949634.0
(22) Date de dépôt: 05.07.2000
(51) Int. Cl.: B01F 17/00

(54) **MICROEMULSION NUTRITIVE PULVERISABLE UTILE COMME ACCELERATEUR DE BIODEGRADATION**
ALS BIODEGRADATIONSBESCHLEUNIGER VERWENDBARE SPRÜHBARE ERNÄHRUNGSMICROEMULSION
NUTRITIONAL SPRAY MICROEMULSION USEFUL AS BIODEGRADATION ACCELERATOR

(30) Priorité: 06.07.1999 FR 9908678
(43) Date de publication de la demande: 11.07.2001
(73) Titulaire: ELF AQUITAINE, 92400 Courbevoie (FR)
(72) Inventeur: TELLIER, Jacques, F-64140 Lons (FR); BASSERES, Anne, F-64320 Bizanos (FR); BROCHETTE, Pascal, F-56200 La Gacilly (FR); ESPERT, Alexandre, F-64000 Pau (FR)
(74) Mandataire: Jolly, Jean-Pierre
(86) Numéro de dépôt international: PCT/FR2000/001920
(87) Numéro de publication internationale: WO 2001/002086

(56) Documents cités:
- EP-A- 0 010 012
- EP-A- 0 120 765
- WO-A-94/05773

## Description

La présente invention concerne un nouvel accélérateur de biodégradation des polluants hydrophobes tels que les hydrocarbures, de type microémulsion eau/huile appelées encore microémulsions nutrientes consommables par les microorganismes.

La formulation de ces microémulsions nutrientes a constitué par le passé une avancée importante pour la mise sur le marché d'additifs utilisables comme accélérateurs de biodégradation. Par exemple, les brevets français 2.490.672 et 2.512.057 décrivent de tels accélérateurs constitués par une phase interne formée d'une solution aqueuse de composés azotés et phosphorés, de préférence des tensio-actifs phosphorés, cette phase étant dispersée dans une phase externe hydrophobe constituée d'une source de carbone assimilable. Dans ces microémulsions, les composés azotés utilisés sont par exemple des aminoacides, des protéines ou de l'urée et les composés phosphorés des tensioactifs de type ester phosphorique d'alkyle ou d'alkényle. La phase externe hydrophobe est constituée par une source de carbone assimilable par les microorganismes comme les huiles végétales ou animales et les acides gras choisis afin d'être compatibles avec les produits à dégrader.

L'efficacité de ces émulsions comme accélérateur de biodégradation a été clairement démontrée dans de nombreuses applications industrielles, notamment le traitement des effets de pollutions marines dues à des déversements accidentels d'hydrocarbures et les procédés de traitement visant la réhabilitation des sols contaminés par des polluants hydrophobes tels que décrits par exemple dans le brevet WO 95/06715.

En dépit des qualités de ces microémulsions nutrientes, leur utilisation reste limitée compte tenu de leur mise en oeuvre difficile, en particulier à basse température, à moins de 10°C.

Les microémulsions décrites dans l'état de la technique sont généralement appliquées par pulvérisation des zones contaminées ou mise en mélange avec des sols pollués. Elles doivent donc présenter une certaine fluidité et une certaine stabilité favorisant de telles applications. Or, ces microémulsions présentent l'inconvénient de se séparer en deux phases non miscibles au voisinage de 0°C. En outre, dès qu'on atteint une température ambiante inférieure à 20°C, ces microémulsions deviennent très visqueuses (plus de 1.000 mPa.s) ce qui rend leur utilisation sur le terrain très délicate voir impossible dans certaines applications comme la pulvérisation.

On a bien entendu tenté de remédier à cet inconvénient majeur par l'adjonction de composés organiques comme les alcools et les éthers de glycol pour diminuer la viscosité de ces microémulsions à ces températures, comme il est également mentionné dans la demande WO 98/07508. Ainsi, en rajoutant de 10 à 15 % de butyléther d'éthylèneglycol, on observe une diminution de la viscosité jusqu'à environ 200 mPa.s à 20° C ce qui permet la pulvérisation du produit avec du matériel conventionnel à cette température ou un épandage plus facile. Cependant, pour appliquer ces microémulsions à plus basse température, en particulier à moins de 10°C, il convient actuellement soit de la stocker à 20°C pour la pulvériser à cette température, soit de la réchauffer sur site pour que la microémulsion se reforme et/ou redevienne fluide. C'est un inconvénient majeur pour l'utilisateur qui comme le produit s'est transformé, n'est plus sûr de la qualité de son produit. On comprend donc pourquoi l'utilisation de cet accélérateur de biodégradation par ailleurs remarquable par son efficacité n'a pu être largement étendue à toutes les applications de biodégradation marines ou terrestres, en particulier dans les pays froids.

La présente invention vise à remédier aux inconvénients rencontrés lors de la mise en oeuvre des microémulsions décrites précédemment. En particulier, la présente invention vise des microémulsions non toxiques, qui sont stables jusqu'à des températures comprises entre -10°C et +50°C et dont la fluidité à + 5°C est suffisante pour qu'il n'y ait aucun problème d'utilisation.

La présente invention a donc pour objet un accélérateur de biodégradation sous forme d'une microémulsion contenant des composés azotés comme par exemple des aminoacides, des protéines ou de l'urée et ses dérivés, des composés phosphorés tensioactifs de type ester phosphorique d'alkyle ou d'alkényle, et des composés choisis dans le groupe des huiles végétales ou animales, ou des acides gras, et d'un fluidifiant, la dite microémulsion étant caractérisée en ce qu'elle comprend de 10 à 35 % en poids d'un tensioactif ester phosphorique mono et/ou dialkylé, ou mono et/ou dialkènylé comprenant un nombre d'atomes de carbone par chaîne alkyle ou alkényle, inférieur à 12, de préférence variant de 3 à 10, le dit ester comprenant en outre de 1 à 10 radicaux alkoxylés, de préférence éthoxylés et/ou propoxylés, avec de 5 à 20 % en poids d'au moins un co-tensioactif, et en ce qu'elle présente une viscosité à 5°C inférieure ou égale à 200 mPa.s.

Dans la présente invention, c'est l'effet combiné de ces tensioactifs dérivés d'esters phosphoriques particuliers avec un co-tensioactif ou un mélange de co-tensioactifs qui permet d'assurer la stabilité de la microémulsion jusqu'à une température très basse, au moins - 10°C et d'utiliser ces produits sans difficulté dans tout environnement froid.

Les cotensioactifs selon l'invention sont choisis parmi les composés présentant une toxicité orale sur le rat telle que la dose léthale pour 50% des microorganismes soit supérieure à 2g/kg.

Les co-tensioactifs seront de préférence choisis comme présentant un point de fusion inférieur à 0°C, et de préférence inférieur à -20°C et/ou une solubilité dans l'eau supérieure à 2g pour 100g d'eau et de préférence supérieure à 10g pour 100g d'eau, ces deux paramètres pouvant être recherchés indépendamment ou simultanément selon les conditions d'utilisation de la microémulsion. On comprend aisément qu'un homme du métier peut être amené à moduler la composition de la microémulsion selon qu'il s'agisse de pulvériser sur une zone polluée en mer ou de traiter un sol à réhabiliter, et selon la température de mise en oeuvre de ces procédés.

Les co-tensioactifs présentant les caractéristiques requises pour faire partie de la microémulsion selon l'invention, seuls ou en mélange, sont choisis parmi les alcools mono et polyhydroxylés comprenant moins de 10 atomes de carbone et leur dérivés éthers, les composés mono et polycarboxyliques, acides ou esters éventuellement mono ou polyhydroxylés, comprenant des chaînes carbonées de C₁ à C₇, les cétones linéaires comprenant au plus 5 atomes de carbone, et les lactones.

Parmi les monoalcools utilisés comme co-tensioactifs de l'invention, on préfère les alkanols de C₂ à C₈, et de préférence de C₂ en C₄.

Parmi les polyalcools, on préfère les diols, notamment les éthylèneglycols substitués ou leurs oligomères, et leurs dérivés éthers comprenant de 4 à 10 atomes de carbone. En particulier, les dérivés éthers préférés sont le butyléther du diéthylèneglycol, le monoéthyléther de l'éthylèneglycol , et le monométhyléther de l'éthylèneglycol.

Parmi les composés monocarboxyliques, éventuellement hydroxylés, sont préférés les acides carboxyliques comprenant de 1 à 4 atomes de carbone et leurs esters dérivant de monoalcools de C₁ à C₅. Dans un mode préféré de l'invention, ces composés monocarboxyliques sont choisis dans le groupe constitué par les acides formique, acétique, butyrique, lactique et leurs esters alkylés comprenant jusqu'à 6 atomes de carbone, le groupement alkyl, le formiate de n-butyle, et le lactate d'éthyle étant préférés.

Parmi les composés dicarboxyliques, éventuellement hydroxylés, sont préférés les diacides carboxyliques comprenant de 3 à 6 atomes de carbone et leurs esters dérivant de monoalcools de C₁ à C₅. Dans un mode préféré de l'invention, ces composés dicarboxyliques sont choisis dans le groupe constitué par les acides malonique et succinique, et leurs esters, de préférence les dialkylmalonates, dont le diéthylmalonate, et les dialkylsuccinates, dont le diméthylsuccinate.

Parmi les cétones de l'invention, on préfère les dialkyles cétones comprenant au plus 6 atomes de carbone et de préférence la méthyléthyl cétone.

Parmi les lactones, la γ-butyrolactone est préférée.

Selon la présente invention, les combinaisons préférées de tensioactifs et de co- tensioactifs sont obtenues par combinaison d'un ester phosphorique d'alkyle, le radical d'alkyle comprenant au plus 6 atomes de carbone, et présentant au plus trois groupements éthoxylés avec des combinaisons d'au moins deux co-tensioactifs

Dans un premier mode de réalisation, ces combinaisons sont obtenues en mélangeant un acide carboxylique avec un composé du groupe constitué par les autres acides carboxyliques éventuellement hydroxylés, leurs esters, les mono et polyalcools et leurs dérivés éthers, les cétones et les lactones. Les combinaisons préférées sont les combinaisons acide acétique - acide butanoique, acide acétique - lactate d'éthyle, acide acétique - éthanol, acide acétique - butyléther de l'éthylèneglycol, acide acétique méthyléthylcétone et acide actétique - γ-butyrolactone,

Dans un second mode de réalisation, ces combinaisons sont obtenues en mélangeant au moins un éther de l'éthylèneglycol ou de ses oligomères, de préférence le butyléther du diéthylèneglycol, avec un alcanol, une cétone comme la méthyléthylcétone ou un ester d'acide monocarboxylique comme le formiate de butyle.

Dans un troisième mode de réalisation, ces combinaisons sont obtenues en mélangeant au moins un ester de diacide carboxylique tel que les alkylsuccinates ou alkylmalonates avec un alcanol ou une cétone. Les combinaisons préférées sont les combinaisons diméthylsuccinate - éthanol et diméthylsuccinate - méthyléthylcétone.

Bien entendu les autres composés de la microémulsion sont choisis pour les composés azotés parmi les aminoacides, l'urée et ses dérivés et pour la phase organique parmi les huiles animales ou végétales et les acides gras, l'acide oléique étant préférée.

Dans la suite de la présente description, des exemples sont donnés pour illustrer l'invention sans en limiter sa portée.

### EXEMPLE I

Le présent exemple vise à montrer que selon les combinaisons tensio-actifs/co-tensioactifs disponibles sur le marché seules les combinaisons synergiques selon l'invention permettent l'obtention de microémulsions stables dans l'intervalle variant de -10°C à +50°C, et fluides à 5°C.

On opère avec trois formules A, B et C dont la composition est donnée dans le tableau I ci-après en pour-cent poids par rapport à l'émulsion.

**TABLEAU I**

| Composants | Formule A | Formule B | Formule C |
|---|---|---|---|
| Tensioactif | 23.7 | 23.4 | 22.4 |
| Co-tensioactif | 10.8 | 12 | 16 |
| eau | 23.6 | 23.3 | 22.3 |
| Urée | 15.7 | 15.4 | 14.4 |
| Acide oléique | 26.2 | 25.9 | 24.9 |

Dans un deuxième tableau, le tableau II ci-après, on va étudier l'influence du couple tensioactif/co-tensioactifs en faisant varier essentiellement la nature du tensioactif, c'est-à-dire en faisant varier la longueur de chaîne alkyle et le nombre de groupements éthoxylés dans l'ester phosphorique. Pour cela, on utilise six tensioactifs différents qu'on caractérise par la longueur de leur chaîne alkyle Cᵢ et par leur nombre de groupement éthoxylés (EO). On examinera la combinaison de ces esters phosphoniques d'alkyles pour les trois formules A,B et C avec des cotensioactifs dont la nature sera précisée. Les échantillons selon l'invention sont appelés Xᵢ et ceux donnés comme échantillons comparatifs sont appelés Tᵢ.

**TABLEAU II**

| Echantilon | Formule | tensioactif | Co-tensioactif | Température en °C | | | Viscosité à 5°C (mPa.s) |
|---|---|---|---|---|---|---|---|
| | | | | +5 | 20 | 50 | |
| T₁ | A | C₁₂-C₁₄ 4EO | BEG | M | M | M | >1000 |
| T₂ | A | C₁₂-C₁₄ 4EO | BDG | M | M | M | >1000 |
| T₃ | C | C₁₂-C₁₄ 4EO | BDG | M | M | M | >1000 |
| T₄ | C | C₁₂-C₁₄ 4EO | Lactate éthyle | M | M | 2φ | >1000 |
| X₁ | C | C₆ 3EO | BDG | M | M | M | 156 |
| X₂ | C | C₆ 3EO | Lactate éthyle | M | M | M | 162 |
| T₃ | C | C₁₀-C₁₄ 4EO | BDG | M | M | M | >1000 |
| T₆ | C | C₁₀-C₁₄ 4EO | lactate éthyle | M | M | M | >1000 |
| T₇ | C | C₁₂-C₁₄ 3EO | BDG | M | M | M | >1000 |
| T₈ | C | C₁₂-C₁₄ 3EO | Lactate éthyle | M | 2φ | M | >1000 |
| T₉ | C | C₁₃6EO | BDG | M | M | M | >1000 |
| T₁₀ | C | C₁₃6EO | Lactate d'éthyle | M | 2φ | M | >1000 |
| T₁₁ | C | C₁₀-C₁₄ 10EO | BDG | M | M | M | >1000 |
| T₁₂ | C | C₁₀-C₁₄ 10EO | Lactate d'éthyle | M | 2φ | M | >1000 |
| T₁₃ | C | C₁₀-C₁₄ 6EO | BDG | M | M | M | >1000 |
| T₁₄ | C | C₁₀-C₁₄ 6EO | Lactate d'éthyle | M | 2φ | M | >1000 |
| T₁₅ | C | C₁₂-C₁₄ 4EO | BDG/Lactate éthyle 9.6/6.4 | M | M | M | >1000 |
| T₁₆ | C | C₁₂-C₁₄ 4EO | BDG/Lactate d'éthyle 8/8 | M | M | 2φ | >1000 |
| BEG = Butyléther de l'éthylèneglycol | | | | | | | |
| BDG = Butyléther du diéthylèneglycol | | | | | | | |
| • (70/30 di/mono) | | | | | | | |
| • M = monophasique | | | | | | | |
| )2φ =diphasique, phase huile en excès | | | | | | | |

On constate d'après ce tableau que l'utilisation de tensio-actifs à chaînes plus courtes (inférieures à C₁₂) permet d'obtenir à la fois des microémulsions stables dans un large domaine de températures, notamment à basses températures, et de faibles viscosités à 5°C.

### EXEMPLE II

Le présent exemple vise à montrer la stabilité des microémulsions selon l'invention lorsqu'on utilise comme tensioactif un ester phosphorique d'alkyle comprenant une longueur de chaîne inférieure à 12, ici 6, et un nombre de groupements éthoxylés inférieur à 10, ici 3 comme dans les échantillons X₁ et X₂ décrits dans le tableau II de l'exemple 1, et que l'on fait varier la nature du ou des co-tensioactifs utilisés.

Comme dans l'exemple I, le tableau III regroupe les mesures de viscosité et de stabilité de l'émulsion pour des échantillons selon l'invention Xi et des échantillons comparatifs Tᵢ.

**TABLEAU III**

| Echantitlon | formule | co-tensioactif | Températures en °C | | | | Viscosité mPa.s à + 5°C |
|---|---|---|---|---|---|---|---|
| | | | -7 | +5 | +20 | +50 | |
| X₁ | C | BDG | M | M | M | M | 158 |
| X₂ | C | Lactate d'éthyle | M | M | M | M | 139 |
| X₃ | A | BDG | M | M | M | M | 158 |
| X₄ | A | Lactate d'éthyle | M | M | M | M | 178 |
| X₅ | B | BDG/Lactate d'éthyle 8/4 | M | M | M | M | 162 |
| T₁₇ | C | Acide lactique | M | M | M | M | >1000 |
| X₈ | B | triéthylecitrate | M | M | M | M | 178 |
| X₇ | B | diéthylemalonate | M | M | M | M | 128 |
| T₁₈ | B | diéthyloxalate | M | M | M | 2φ | >1000 |
| X₈ | B | diméthylsuccinate | M | M | M | M | 146 |
| X₉ | B | éthylformiate | M | M | M | M | 139 |
| T₁₉ | B | propylformiate | 2φ | M | M | 2φ | 131 |
| X₁₀ | C | isopropylformiate | M | M | M | M | 95 |
| T₂₀ | B | butylformiate | 2φ | 2φ | 2φ | M | - |
| T₂₁ | B | éthanol | 2φ | 2φ | M | 2φ | - |
| T₂₂ | B | isopropanol | 2φ | 2φ | M | 2φ | - |
| T₂₃ | B | isobutanol | 2φ | 2φ | M | 2φ | - |
| X₁₁ | B | terbutanol | M | M | M | M | - |
| T₂₄ | B | γ-butyrolactone | M | M | M | M | >1000 |
| T₂₅ | B | MEG | 2φ | M | M | M | >1000 |
| T₂₆ | B | EEG | M | M | M | M | >1000 |
| X₁₂ | B | MEC | M | M | M | M | 131 |
| X₁₃ | B | Acide éthanoique | M | M | M | M | 162 |
| X₁₄ | B | Acide butanoique | M | M | M | M | 119 |
| X₁₅ | B | BDG/MEC 9.6/2.4 | M | M | M | M | 148 |
| X₁₆ | B | BDG/éthanol 9/3 | M | M | M | M | 106 |
| X₁₇ | B | BDG/ butytformiate 9/3 | M | M | M | M | 131 |
| X₁₈ | B | BOG/ Ac.acétique 6/6 | M | M | M | M | 151 |
| X₁₉ | B | Ac.acétique/Ac butanoique 6/6 | M | M | M | M | 147 |
| X₂₀ | B | Ac.acétique/tactateéthyle 8/4 | M | M | M | M | 131 |
| X₂₁ | B | Ac.acétique/éthanol 8/4 | M | M | M | M | 131 |
| X₂₂ | B | Ac.acétique/ γ-butyrolactone 8.5/3.5 | M | M | M | M | 200 |
| X₂₃ | B | Ac.acétique/MEC 8/4 | M | M | M | M | 131 |
| X₂₄ | B | diméthylsuccinate/éthanol 8/4 | M | M | M | M | 140 |
| X₂₅ | B | diméthylsuccinate/ MEC 8/4 | M | M | M | M | 132 |
| T₂₇ | B | MEC/ γ-butyrolactone 4.2.5/7.8 | M | M | M | M | >1000 |
| MEC =mèthylèthylcétone | | | | | | | |
| EEG =éthyléthylène glycol | | | | | | | |
| MEG = monoéthylèneglycol | | | | | | | |

D'après ce tableau, on constate que seuls les échantillons monophasiques sur la plage de température -7°C à +50°C, et de viscosité mesurable à +5°C suffisamment faible, inférieure à 200 mPa.s pour leur permettre d'être fluide à basse température, sont considérés comme des échantillons Xi. On notera en outre que certains co-tensioactifs utilisés seuls dans certaines formules A, B ou C peuvent ne pas être considérés comme utilisables à basses températures du fait de leur viscosité excessive, mais peuvent être jugés très intéressants en combinaison avec un autre co-tensioactif selon l'invention.

### EXEMPLE III

Le présent exemple vise à décrire l'effet accélérateur de biodégradabilité des microémulsions selon l'invention au regard des hydrocarbures sur un pilote de radiorespiromètrie.

On opère à l'aide d'un appareillage de laboratoire dit radiorespiromètre tel que décrit par F. Bruchon, A.Basséres et J.C.Bertrand Biotechnol. Lett. (1996), 18(1), 111-16. Il permet de suivre en continu la minéralisation des hydrocarbures radioactifs, tout en évaluant les pertes abiotiques sur chaque culture ce qui évite d'incuber des témoins stériles.

On introduit 100 ml du milieu réactionnel constitué par de l'eau de mer synthétique, une flore bactérienne et du phénanthrène marqué en ¹⁴C, difficilement biodégradable, à 100mg/l, dans des flacons de cultures ou erlenmeyers de 250ml équipés de deux torions latéraux l'un étant raccordé à l'arrivée d'oxygène et l'autre étant connecté à un piège à hydrocarbures constitué par des colonnes de type ORBO-43 vendues par SUPELCO. On ajuste le débit d'aération en oxygène à 5ml/mn, et on maintient le milieu sous agitation avec un agitateur va-et-vient à 80 oscillations par minute. Ces réacteurs ainsi agités sont placés à l'obscurité pour incubation à 20°C pendant un mois. Les hydrocarbures évaporés s'absorbent sur les pièges à hydrocarbures. Le flux d'air entraîne le CO₂ marqué en ¹⁴C · issu de la minéralisation du phénanthrène vers un piège à CO₂ constitué par une solution de soude 4N.

Pendant cette incubation d'un mois, pour mesurer la cinétique de minéralisation, on prélève tous les deux ou trois jours selon les résultats obtenus, trois échantillons aliquotes de 0.5ml de la solution de soude 4N qui sont mis à compter dans 10 ml de Coktail Hionic-Fluor de chez Packard.

La flore bactérienne utilisée pour tester l'efficacité des microémulsions, ou plutôt du couple tensioactif /co-tensioactif(s) est une flore naturelle complexe d'origine marine. Avant de démarrer les essais, on réactive cette flore maintenue à - 80°C sur un bouillon riche Marine Agar 2216 de chez Difco pendant huit heures, puis on la pré-cultive sur une eau de mer synthétique de type Instant Ocean à 33g/l, enrichie à 50mg/l en pétrole brut Arabe léger pendant deux jours en présence de nutriments azotés et phosphorés, de chlorure d'ammonium et d'hydrogènophosphate de potassium en concentrations telles que les rapports C/N/P sont égaux à 106/16/1.

Le phénanthrène marqué utilisé est du 9-¹⁴C phénanthrène, produit Sigma 31,528-1.

Les tests sont effectués sur 9 réacteurs placés en série contenant tous le même milieu réactionnel constitué par l'eau de mer synthétique, le phénanthrène marqué à 100mg/l et la Flore bactérienne activée à 10%v/v, et un tampon pH neutre, le TRIS à 6g/l, ces concentrations étant rapportées au milieu réactionnel. On introduit dans huit de ces réacteurs différentes microémulsions selon l'invention et selon l'art antérieur. Les concentrations des microémulsions dans le milieu réactionnel au début des essais sont de 10 mg/l. Ces microémulsions correspondent aux microémulsions décrites dans les exemples I et II. Le détail du contenu de chaque réacteur est donné ci - après.
Réacteur 1 : milieu réactionnel seul = témoin
Réacteur 2 : milieu réactionnel + X₁₅
Réacteur 3 : milieu réactionnel + X₁₆
Réacteur 4 : milieu réactionnel + X₁₈
Réacteur 5 : milieu réactionnel + X₁₉
Réacteur 6 : milieu réactionnel + X₂₀
Réacteur 7 : milieu réactionnel + X₂₃
Réacteur 8 : milieu réactionnel + X₅
Réacteur 9 : milieu réactionnel + T₁

Les résultats chiffrés exprimés en % des taux de minéralisation du phénanthrène en présence de ces différentes microémulsions sont rassemblés dans le tableau IV ci-après.

**TABLEAU IV**

| Taux minéralisation | Témoin | X₁₅ | X₁₆ | X₁₈ | X₁₉ | X₂₀ | X₂₃ | X₅ | T₁ |
|---|---|---|---|---|---|---|---|---|---|
| 0 | 0 | 0 | 0 | 0 | 0 | 0 | 0 | 0 | 0 |
| 8 jours | 5 | 0.1 | 0.8 | 0.1 | 1.6 | 2.19 | 1.3 | 0.05 | 5 |
| 14 jours | 12.9 | 4.1 | 43.7 | 1.6 | 1.5 | 51.2 | 9.4 | 2.8 | 29.1 |
| 20 jours | 18 | 24.8 | 68.4 | 1.8 | 1.5 | 71 | 44.2 | 34.1 | 42 |
| 25 jours | 22.6 | 55 | 76.4 | 12.7 | 1.6 | 78.2 | 56.8 | 56.4 | 51.9 |

On constate une meilleure minéralisation du phénanthrène en présence des microémulsions X₁₆ et X₂₀ . Pour certaines autres formulations selon l'invention, on observe un certain temps de latence avant que la minéralisation ne se produise.

Les microémulsions X₁₈ et X₁₉ sont moins favorables car plus biodégradables que le phénanthrène par les bactéries : ils sont biodégradés avant le phénanthrène.

## Revendications

1. Microémulsion utilisée comme accélérateur de biodégradation, stable entre -10°C et +50°C, contenant des composés azotés comme par exemple des aminoacides, des protéines ou de l'urée et des composés phosphorés tensioactifs de type ester phosphorique d'alkyle ou d'alkényle, et des composés choisis dans le groupe des huiles végétales ou animales, ou des acides gras, et un fluidifiant, la dite microémulsion étant **caractérisée en ce qu'**elle comprend de 10 à 35 % en poids d'un tensioactif ester phosphorique mono et/ou dialkylé, ou mono et/ou dialkènylé, comprenant un nombre d'atomes de carbone par chaîne alkyle ou alkényle, inférieur à 12, le dit ester comprenant en outre de 1 à 10 radicaux alkoxylés, avec de 3 à 20 % en poids d'au moins un co-tensioactif, et **en ce qu'**elle présente une viscosité à 5°C inférieure ou égale à 200 mPa.s.

2. Microémulsion selon la revendication 1, caractérisée en ce l'ester phosphorique mono et/ou dialkylé, ou mono et/ou dialkènylé, comprend un nombre d'atomes de carbone par chaîne alkyle ou alkènyle compris entre 4 et 10.

3. Microémulsion selon l'une des revendications 1 et 2, **caractérisée en ce que** les radicaux alkoxylés de l'ester phosphorique mono et/ou dialkylé, ou mono et/ou dialkènylé, sont des radicaux éthoxylés et/ou propoxylés.

4. Microémulsion selon les revendications 1 à 3, **caractérisée en ce que** les co-tensioactifs présentent une toxicité orale sur le rat telle que la dose léthale pour 50% des organismes soit supérieure à 2g/kg.

5. Microémulsion selon les revendications 1 à 4, **caractérisée en ce que** le co-tensioactif présente un point de fusion inférieur à 0°C, et dé préférence inférieur à -20°C, et/ou une solubilité dans l'eau supérieure à 2g pour 100g à 20°C, et de préférence supérieure à 10g pour 100g d'eau.

6. Microémulsion selon les revendications 1 à 5, **caractérisée en ce que** les co-tensioactifs, pris seuls ou en mélange, sont choisis parmi les alcools mono et polyhydroxylés comprenant moins de 10 atomes de carbone et leur dérivés éthers, les composés mono et polycarboxyliques, acides ou esters éventuellement mono ou polyhydroxylés, comprenant des chaînes carbonées de C₁ à C₇, et les cétones linéaires et cycliques comprenant au plus 5 atomes de carbone et les lactones.

7. Microémulsion selon les revendications 1 à 6, **caractérisée en ce que** les monoalcools sont des alkanols de C₂ à C₈, et de préférence de C₂ à C₄.

8. Microémulsion selon les revendications 1 à 7, **caractérisée en ce que** les polyalcools sont des diols, notamment des éthylèneglycols substitués ou leurs oligomères, et leurs dérivés éthers comprenant de 4 à 10 atomes de carbone.

9. Microémulsion selon la revendication 8, **caractérisée en ce que** les dérivés éthers de l'éthylèneglycol ou de ses oligomères sont choisis parmi le butyléther du diéthylèneglycol, le monoéthylether de l'éthylèneglycol et le diéthyléther de l'éthylèneglycol.

10. Microémulsion selon les revendications 1 à 9, **caractérisée en ce que** les composés monocarboxyliques, éventuellement hydroxylés, sont choisis parmi les acides carboxyliques comprenant de 1 à 4 atomes de carbone et leurs esters dérivant de monoalcools de C₁ à C₅.

11. Microémulsion selon la revendication 10, **caractérisée en ce que** les composés monocarboxyliques sont choisis dans le groupe constitué par les acides formique, acétique, butyrique et lactique, leurs esters d'alkyle comprenant jusqu'à 6 atomes de carbone dans le groupement alkyl, dont le formiate de n-butyle, et le lactate d'éthyle.

12. Microémulsion selon les revendications 1 à 11, **caractérisée en ce que** les composés dicarboxyliques, éventuellement hydroxylés, sont choisis parmi les diacides carboxyliques comprenant de 3 à 6 atomes de carbone et leurs esters dérivant de monoalcools de C₁ à C₅.

13. Microémulsion selon la revendication 12, **caractérisée en ce que** les composés dicarboxyliques sont choisis dans le groupe constitué par les acides malonique et succinique, les dialkylmalonates, de préférence le diéthylmalonate, et les dialkylsuccinates, de préférence le diméthylsuccinate.

14. Microémulsion selon les revendications 1 à 13, **caractérisée en ce que** les cétones sont des dialkylcétones comprenant au plus 6 atomes de carbone et de préférence la méthyléthylcétone.

15. Microémulsion selon les revendications 1 à 14, **caractérisée en ce que** les lactones comprennent au plus 6 atomes de carbone, la γ-butyrolactone étant préférée.

16. Microémulsion selon les revendications 1 à 15, **caractérisée en ce que** les combinaisons de tensioactifs et de co-tensioactifs sont obtenues par combinaison d'un ester phosphorique d'alkyle, le radical alkyle comprenant au plus 6 atomes de carbone et présentant au plus trois groupements éthoxylés avec des combinaisons d'au moins deux co-tensioactifs.

17. Microémulsion selon les revendications 1 à 16, **caractérisée en ce que** les combinaisons de co-tensioactifs sont obtenues en mélangeant un acide carboxylique avec un composé du groupe constitué par les autres acides carboxyliques éventuellement hydroxylés, leurs esters, les mono et polyalcools, et leurs dérivés éthers, les cétones et les lactones.

18. Microémulsion selon la revendication 17, **caractérisée en ce que** les combinaisons sont choisies parmi les combinaisons acide acétique - acide butanoïque, acide acétique - lactate d'éthyle, acide acétique - éthanol, acide acétique - butyléther du diéthylèneglycol, acide acétique - méthyléthylcétone et acide acétique -γ-butyrolactone.

19. Microémulsion selon les revendications 1 à 16, **caractérisée en ce que** les combinaisons de co-tensioactifs sont obtenues en mélangeant au moins un éther de l'éthylèneglycol ou de ses oligomères, de préférence le butyléther du diéthylèneglycol, avec un alcanol, une cétone cornme la méthyléthylcétone ou un ester d'acide monocarboxylique comme le formiate de butyle.

20. Microémulsion selon les revendications 1 à 16, **caractérisée en ce que** les combinaisons de co-tensioactifs sont obtenues en mélangeant au moins un ester de diacide carboxylique tel que les alkylsuccinates ou les alkylmalonates avec un alcanol ou une cétone.

21. Microémulsion selon la revendication 20, **caractérisée en ce que** les combinaisons sont choisies parmi les combinaisons diméthylsuccinate -éthanol et diméthylsuccinate - méthyléthylcétone.

## Patentansprüche

1. Als Biodegradationsbeschleuniger verwendete, zwischen -10°C und +50°C stabile Microemulsion, die Stickstoffverbindungen wie zum Beispiel Aminosäuren, Proteine oder Harnstoff und oberflächenaktive Phosphorverbindungen vom Typ eines Alkyl- oder Alkenyl-Phosphoresters, und Verbindungen, die aus der Gruppe der pflanzlichen oder tierischen Öle oder der Fettsäuren ausgewählt sind, und einen Verflüssiger enthält, wobei die besagte Microemulsion **dadurch gekennzeichnet ist, daß** sie 10 bis 35 Gew.-% eines oberflächenaktiven Mono- und/oder Di-Alkyl- oder Mono- und/oder Di-Alkenyl-Phosphorester mit einer Zahl an Kohlenstoffatomen pro Alkyl- oder Alkenylkette von weniger als 12, wobei der besagte Ester außerdem 1 bis 10 Alkoxylreste umfaßt, zusammen mit 3 bis 20 Gew.-% mindestens eines co-oberflächenaktiven Mittels umfaßt, und daß sie eine Viskosität bei 5°C von weniger oder gleich 200 mPa·s zeigt.

2. Microemulsion gemäß Anspruch 1, **dadurch gekennzeichnet, daß** der Mono- und/oder Di-Alkyl- oder Mono- und/oder Di-Alkenyl-Phosphorester eine Zahl an Kohlenstoffatomen pro Alkyl- oder Alkenyl-Kette von 4 bis 10 aufweist.

3. Microemulsion gemäß einem der Ansprüche 1 und 2, **dadurch gekennzeichnet, daß** die Alkoxylreste des Mono- und/oder Di-Alkyl- oder Mono- und/oder Di-Alkenyl-Phosphoresters Ethoxylund/oder Proproxylreste sind.

4. Microemulsion gemäß den Ansprüchen 1 bis 3, **dadurch gekennzeichnet, daß** die co-oberflächenaktiven Mittel eine orale Toxizität auf die Ratte derart zeigt, daß die letale Dosis für 50% der Organismen mehr als 2g/kg ist.

5. Microemulsion gemäß den Ansprüchen 1 bis 4, **dadurch gekennzeichnet, daß** das co-oberflächenaktive Mittel einen Erweichungspunkt von weniger als 0°C und vorzugsweise von weniger als -20°C, und/oder eine Löslichkeit in Wasser von mehr als 2g pro 100g bei 20°C und vorzugsweise von über 10g pro 100g Wasser zeigt.

6. Microemulsion gemäß den Ansprüchen 1 bis 5, **dadurch gekennzeichnet, daß** die co-oberflächenaktiven Mittel, allein oder in Mischung, ausgewählt sind unter mono- und polyhydroxylierten Alkoholen mit mindestens 10 Kohlenstoffatomen sowie deren Esterderivaten, den mono- und polycarboxylierten Säureoder Esterverbindungen, die ggf. mono- oder polyhydroxyliert sind, mit Kohlenstoffketten von C₁ bis C₇, sowie den linearen und cyclischen Ketonen mit höchstens 5 Kohlenstoffatomen, und den Laktonen.

7. Microemulsion gemäß den Ansprüchen 1 bis 6, **dadurch gekennzeichnet, daß** die Monoalkohole C₂- bis C₈- und vorzugsweise C₂- bis C₄-Alkohole sind.

8. Microemulsion gemäß den Ansprüchen 1 bis 7, **dadurch gekennzeichnet, daß** die Polyalkohole Diole, insbesondere substituierte Ethylenglykole, oder deren Oligomere, sowie deren Etherderivate mit 4 bis 10 Kohlenstoffatomen sind.

9. Microemulsion gemäß Anspruch 8, **dadurch gekennzeichnet, daß** die Etherderivate von Ethylenglycol oder deren Oligomere ausgewählt sind aus dem Butylether von Diethylenglykol, dem Monoethylether von Ethylenglykol, und dem Diethylether von Ethylenglykol.

10. Microemulsion gemäß den Ansprüchen 1 bis 9, **dadurch gekennzeichnet, daß** die monocarboxylierten, ggf. hydroxilierten Verbindungen ausgewählt sind unter den Carboxylsäuren mit 1 bis 4 Kohlenstoffatomen und deren Estern, die von C₁- bis C₅-Monoalkoholen stammen.

11. Microemulsion gemäß Anspruch 10, **dadurch gekennzeichnet, daß** die monocarboxylierten Verbindungen ausgewählt sind aus der Gruppe, die aus Ameisensäure, Essigsäure, Buttersäure und Milchsäure, deren Alkylestern mit bis zu 6 Kohlenstoffatomen in der Alkylgruppe, dessen n-Butyl-Formiat und Ethyl-Lactat besteht.

12. Microemulsion gemäß den Ansprüchen 1 bis 11, **dadurch gekennzeichnet, daß** die dicarboxylierten, ggf. hydroxilierten Verbindungen ausgewählt sind aus Dicarboxylsäuren mit 3 bis 6 Kohlenstoffatomen und deren Estern, die von C₁- bis C₅-Monoalkoholen stammen.

13. Microemulsion gemäß Anspruch 12, **dadurch gekennzeichnet, daß** die dicarboxylierten Verbindungen ausgewählt sind aus der Gruppe, die aus Malonsäure und Bernsteinsäure, den Dialkylmalonaten, vorzugsweise Diethylmalonat, und den Dialkylsuccinaten, vorzugsweise Dimethylsuccinat, besteht.

14. Microemulsion gemäß den Ansprüchen 1 bis 13, **dadurch gekennzeichnet, daß** die Ketone Dialkylketone mit höchstens 6 Kohlenstoffatomen sind, vorzugsweise das Methylethylketon.

15. Microemulsion gemäß den Ansprüchen 1 bis 14, **dadurch gekennzeichnet, daß** die Laktone höchstens 6 Kohlenstoffatome umfassen, wobei γ-Butyrolakton bevorzugt ist.

16. Microemulsion gemäß den Ansprüchen 1 bis 15, **dadurch gekennzeichnet, daß** die Kombinationen der oberflächenaktiven Mittel und der co-oberflächenaktiven Mittel erhalten wurden durch Kombination eines Alkyl-Phosphoresters, wobei der Alkylrest höchstens 6 Kohlenstoffatome umfaßt und höchstens 3 Ethoxylgruppen zeigt, mit Kombinationen von mindestens zwei co-oberflächenaktiven Mitteln.

17. Microemulsion gemäß den Ansprüchen 1 bis 16, **dadurch gekennzeichnet, daß** die Kombinationen der co-oberflächenaktiven Mittel erhalten wurden durch Mischen einer Carboxylsäure mit einer Verbindung aus einer Gruppe, die aus anderen, ggf. hydroxylierten Carboxylsäuren, deren Estern, Mono- und Polyalkoholen und deren Etherderivaten, Ketonen und Laktonen besteht.

18. Microemulsion gemäß Anspruch 17, **dadurch gekennzeichnet, daß** die Kombinationen ausgewählt sind unter den Kombinationen Essigsäure/Buttersäure, Essigsäure/Ethyllactat, Essigsäure/Ethanol, Essigsäure/Diethylenglycol-butylether, Essigsäure/Methylethylketon und Essigsäure/γ-Butyrolakton.

19. Microemulsion gemäß den Ansprüchen 1 bis 16, **dadurch gekennzeichnet, daß** die Kombinationen der co-oberflächenaktiven Mittel erhalten wurden durch Mischen mindestens eines Ethers von Ethylenglycol oder von dessen Oligomeren, vorzugsweise dem Butylether von Diethylenglycol, mit einem Alkanol, einem Keton wie Methylethylketon, oder einem Monocarboxylsäureester wie Butylformiat.

20. Microemulsion gemäß den Ansprüchen 1 bis 16, **dadurch gekennzeichnet, daß** die Kombinationen der co-oberflächenaktiven Mittel erhalten wurden durch Mischen mindestens eines Dicarbonsäureesters wie den Alkylsuccinaten oder den Alkylmalonaten mit einem Alkanol oder einem Keton.

21. Microemulsion gemäß Anspruch 20, **dadurch gekennzeichnet, daß** die Kombinationen ausgewählt wurden unter den Kombinationen Dimethylsuccinat/Ethanol und Dimethylsuccinat/Methylethylketon.

## Claims

1. A microemulsion used as a biodegradation accelerator, stable between -10°C and +50°C, containing nitrogenous compounds such as for example amino acids, proteins or urea and phosphorous surfactant compounds of the alkyl or alkenyl phosphoric ester type, and compounds selected from the group comprising vegetable or animal oils or fatty acids, and a plasticizer, said microemulsion being **characterised in that** it comprises 10 to 35% by weight of a mono- and/or dialkyl- or mono- and/or dialkenylphosphoric ester surfactant, comprising a number of carbon atoms per alky or alkenyl chain which is lower than 12, said ester further comprising 1 to 10 alkoxylated radicals, with 3 to 20% by weight of at least one cosurfactant, and **in that** it has a viscosity at 5°C which is less than or equal to 200 mPa·s.

2. A microemulsion according to claim 1, **characterised in that** the mono- and/or dialkyl- or mono- and/or dialkenylphosphoric ester comprises a number of carbon atoms per alkyl or alkenyl chain which is between 4 and 10.

3. A microemulsion according to one of claims 1 and 2, **characterised in that** the alkoxylated radicals of the mono- and/or dialkyl-, or mono- and/or dialkenylphosphoric ester are ethoxylated and/or propoxylated radicals.

4. A microemulsion according to claims 1 to 3, **characterised in that** the cosurfactants have an oral toxicity in rats such that the lethal dose for 50% of the organisms is greater than 2 g/kg.

5. A microemulsion according to claims 1 to 4, **characterised in that** the cosurfactant has a melting point lower than 0°C, and preferably lower than -20°C, and/or a solubility in water greater than 2 g per 100 g at 20°C, and preferably greater than 10 g per 100 g of water.

6. A microemulsion according to claims 1 to 5, **characterised in that** the cosurfactants, alone or in a mixture, are selected from among mono- and polyhydroxylated alcohols comprising fewer than 10 carbon atoms and their ether derivatives, mono- and polycarboxylic compounds, acids or esters which are optionally mono- or polyhydroxylated, comprising C₁ to C₇ carbon chains, and linear and cyclic ketones comprising at most 5 carbon atoms, and lactones.

7. A microemulsion according to claims 1 to 6, **characterised in that** the monoalcohols are C₂ to C₈, preferably C₂ to C₄, alkanols.

8. A microemulsion according to claims 1 to 7, **characterised in that** the polyalcohols are diols, in particular substituted ethylene glycols or their oligomers, and their ether derivatives comprising 4 to 10 carbon atoms.

9. A microemulsion according to claim 8, **characterised in that** the ether derivatives of ethylene glycol or its oligomers are selected from among diethylene glycol butyl ether, ethylene glycol monoethyl ether and ethylene glycol diethyl ether.

10. A microemulsion according to claims 1 to 9, **characterised in that** the monocarboxylic, optionally hydroxylated, compounds are selected from among carboxylic acids comprising 1 to 4 carbon atoms and their esters derived from C₁ to C₅ monoalcohols.

11. A microemulsion according to claim 10, **characterised in that** the monocarboxylic compounds are selected from the group consisting of formic, acetic, butyric and lactic acids and their alkyl esters comprising up to 6 carbon atoms in the alkyl group, for instance n-butyl formate and ethyl lactate.

12. A microemulsion according to claims 1 to 11, **characterised in that** the dicarboxylic, optionally hydroxylated, compounds are selected from among carboxylic diacids comprising 3 to 6 carbon atoms and their esters derived from C₁ to C₅ monoalcohols.

13. A microemulsion according to claim 12, **characterised in that** the dicarboxylic compounds are selected from the group consisting of malonic and succinic acids, dialkyl malonates, preferably diethyl malonate, and dialkyl succinates, preferably dimethyl succinate.

14. A microemulsion according to claims 1 to 13, **characterised in that** the ketones are dialkyl ketones comprising at most 6 carbon atoms and preferably methyl ethyl ketone.

15. A microemulsion according to claims 1 to 14, **characterised in that** the lactones comprise at most 6 carbon atoms, γ-butyrolactone being preferred.

16. A microemulsion according to claims 1 to 15, **characterised in that** the combinations of surfactants and cosurfactants are obtained by combining an alkyl phosphoric ester, the alkyl radical comprising at most 6 carbon atoms and having at least three ethoxylated groups with combinations of at least two cosurfactants.

17. A microemulsion according to claims 1 to 16, **characterised in that** the cosurfactant combinations are obtained by mixing a carboxylic acid with a compound of the group consisting of other, optionally hydroxylated carboxylic acids, their esters, mono- and polyalcohols and their ether derivatives, ketones and lactones.

18. A microemulsion according to claim 17, **characterised in that** the combinations are selected from among the following combinations: acetic acid/butanoic acid, acetic acid/ethyl lactate, acetic acid/ethanol, acetic acid/diethylene glycol butyl ether, acetic acid/methyl ethyl ketone and acetic acid/γ-butyrolactone.

19. A microemulsion according to claims 1 to 16, **characterised in that** the cosurfactant combinations are obtained by mixing at least one ether of ethylene glycol or its oligomers, preferably diethylene glycol butyl ether, with an alkanol, a ketone such as methyl ethyl ketone or a monocarboxylic acid ester such as butyl formate.

20. A microemulsion according to claims 1 to 16, **characterised in that** the cosurfactant combinations are obtained by mixing at least one carboxylic diacid ester such as alkyl succinates or alkyl malonates with an alkanol or a ketone.

21. A microemulsion according to claim 20, **characterised in that** the combinations are selected from among the combinations dimethyl succinate/ethanol and dimethyl succinate/methyl ethyl ketone.
